# EUROPÄISCHE PATENTSCHRIFT

(11) **EP 0 777 440 B1**
(45) Veröffentlichungstag und Bekanntmachung des Hinweises auf die Patenterteilung: **19.01.2005**
(21) Anmeldenummer: 96919609.6
(22) Anmeldetag: 22.05.1996
(51) Int. Cl.: A61B 6/04, A61B 6/08

(54) **VORRICHTUNG ZUR COMPUTERTOMOGRAPHIE-DURCHLEUCHTUNG FÜR INTERVENTIONEN**
DEVICE FOR COMPUTER TOMOGRAPHY TRANSILLUMINATION FOR TREATMENT
DISPOSITIF DE RADIOSCOPIE-TOMOGRAPHIE ASSISTEE PAR ORDINATEUR UTILISEE POUR DES INTERVENTIONS

(30) Priorität: 23.05.1995 DE 19520017; 20.05.1996 DE 29609826 U
(43) Veröffentlichungstag der Anmeldung: 11.06.1997
(73) Patentinhaber: Rogalla, Patrik, Dr., 10829 Berlin (DE); Mutze, Sven, Dr., 15566 Schöneiche (DE)
(72) Erfinder: Rogalla, Patrik, Dr., 10829 Berlin (DE); Mutze, Sven, Dr., 15566 Schöneiche (DE)
(74) Vertreter: Wehlan, Helmut, Dr.
(86) Internationale Anmeldenummer: PCT/DE1996/000944
(87) Internationale Veröffentlichungsnummer: WO 1996/037149

(56) Entgegenhaltungen:
- DE-A- 4 202 302
- US-A- 3 588 500
- US-A- 4 117 337
- US-A- 4 360 028
- US-A- 5 199 060

## Beschreibung

Die Erfindung bezieht sich auf eine Vorrichtung an Computertomographen (CT), insbesondere zur Computertomographie-Durchleuchtung für Interventionen. Sie betrifft die Einstellung eines Patiententisches, die Fixierung seiner Position sowie die Auslösung eines Lichtvisiers und eines Einzelscans nach mechanischer Entkopplung des CT vom zentralen Steuerungssystem.

Die Computertomographie hat die medizinische Diagnostik außerordentlich bereichert und nicht nur zahlreiche strahlenexponierende Röntgenuntersuchungen, wie Enzephalographie oder Angiographie, überflüssig gemacht, sondern auch die mit ihr gewonnenen Schichtbilder für die Planung und

Kontrolle der Strahlentherapie von Tumoren nutzbar gemacht. Zahlreiche pathologische Prozesse im gesamten Körperstamm, wie z.B. Karzinome oder vergrößerte Lymphknoten, können nur durch eine Gewebeentnahme klassifiziert und anschließend therapiert werden. Derartige Biopsien lassen sich unter anderem mit Hilfe der Computertomographie durchführen. Dabei wird die Position einer in den Körper eingeführten Nadel durch Anfertigen eines einzelnen Schnittbildes in der Ebene der Nadel dargestellt.

Nach entsprechender Vorbereitung des Patienten, d.h. Lagerung, Desinfizieren der Haut und örtlicher Betäubung an der Stelle des Einstiches, wird üblicherweise - außerhalb der Gantry - die Biopsienadel oberflächlich unter die Haut eingeführt und anschließend der Patiententisch zur Durchführung des ersten Kontrollscans in die Gantry gefahren. Die Steuerung dieses Vorganges erfolgt von der konsole des CT, welche aus Strahlenschutzgründen außerhalb des Untersuchungsraumes steht. Das medizinische Personal verlässt den Untersuchungsraum bei Aufnahme einer Schicht. Nachdem auf dem Monitor das Schnittbild erscheint, wird der Patient aus der Gantry gefahren. Nach ggf. erforderlicher Lagekorrektur der Nadel erfolgt eine erneute Aufnahme, nachdem Tisch und Patient von der Konsole aus in die Gantry gefahren werden. Die gesamte Prozedur - Bewegen des Patienten aus der Gantry und erneutes Hineinfahren - geht mit einem hohen Zeitaufwand einher, und der Patient muss während der gesamten Untersuchung ruhig und bewegungsfrei liegen bleiben.

Es sind verschiedene Lösungen vorgeschlagen worden, die sich mit der Einstellung einer möglichst genauen Position des Patienten bzw. bestimmter Körperteile oder von Patiententischen befassen, z.B.:
- Positionieren eines Patienten bei einer medizinischen Panorama-Röntgenaufnahmeeinrichtung mit Hilfe eines Sensors zum Ermitteln der Relativlage eines zu untersuchenden Objekts zu der Röntgenaufnahmeeinrichtung, wobei die ermittelten Daten mit denen der Relativlage einer Tomographiezone zu der Einrichtung verglichen werden ( DE 3808009; DE = Deutsche Patent- bzw. Offenlegungsschrift)
- eine feststehend angeordnete Patientenhalterung, während das Gehäuse des Computertomographen (CT) mittels Antriebs- und Führungseinrichtungen linear geführt verschiebbar ist (GM 9218322; GM = Deutsches Gebrauchsmuster)
- eine motorisch angetriebene und rechnergesteuerte Patientenliege, die in das CT-Gehäuse einfahrbar ist
- eine synchrone Bewegung des Patienten mit der Aufnahme sukzessiver Scans, ausgehend von einer Initialposition, z.B. bei Untersuchungen des Schädelbereichs (EP 579036; EP = Europäisches Patent)
- ein CT-Gehäuse zur Aufnahme der Patientenliege, wobei - zur Vermeidung von Informationsverlusten zwischen Diagnose und Eingriff - die Patientenliege zugleich als Operationstisch ausgebildet ist (DE 4202302)
- ärztliche oder zahnärztliche Untersuchungseinrichtungen, etwa zur Kiefergelenk-Aufnahme (DE 3937077) oder zur CT des Knies nach Fixierung des Unterschenkels (DE 3609535)
- CT mit Mitteln zur Erzeugung eines Schattenbildes, das synchron mit der jeweiligen Position des Meßsystems zur Patientenliege auf einem Monitor dargestellt wird (DE 4218637, US (= US-Patent) 5373543).

Dokument US-A-4 360 028 offenbart eine Schädel-Einführung einer Nadel durch Computer tomographie.

Der Erfindung liegt die Aufgabe zugrunde, eine Vorrichtung zu entwickeln, durch die zur Computertomographie-Durchleuchtung für Interventionen eine exakte Einstellung der Position des Patienten erreicht und die Anzahl von Scans zum Suchen der Nadel verringert werden kann.

Die Aufgabe wurde durch die Kombination eines schwimmenden Patiententisches mit einer Schnittbildmodalität für Biopsien unter CT-Sichtkontrolle gelöst. Die erfindungsgemäße Vorrichtung besteht darin, dass sie einen mit einem Handgriff versehenen Patiententisch eines Computertomographen zur mechanischen Einstellung einer beliebigen Position des Tisches nach Entkopplung vom zentralen Steuerungssystem enthält und Fußschalter zum Auslösen eines Lichtvisiers zum einen und eines Einzelscans zum anderen. Der neu angebrachte Handgriff am Patiententisch macht es möglich, den Tisch nach mechanischer Entkopplung von der zentralen Steuerung beliebig zu positionieren. Die Fußschalter werden im Untersuchungsraum installiert. Sie können sowohl das Lichtvisier als auch einen Einzelscan auslösen.

Als *schwimmender Patiententisch* wird eine Patientenliege bezeichnet, die mit nur geringer mechanischer Kraftanwendung durch den Untersucher zu jedem Zeitpunkt der Untersuchung frei entlang einer Achse bewegt werden kann.

Unter *Schnittbildmodalität* wird eine Untersuchungstechnik verstanden, die zur Visualisierung eines Untersuchungsobjektes Schnittbilder in einer Ebene mit einer definierten Schichtdicke erzeugt. Zu den Schnittbildmodalitäten zählen die Computertomographie, der Ultraschall oder die Kemspintomographie.

Die Installation des Handgriffs am Patiententisch macht es möglich, während des Eingriffs jede beliebige Tischposition per Hand einzustellen. Die digital angezeigte Tischposition stimmt stets mit der wahren Position überein. Das Lichtvisier wird über einen zusätzlich installierten Fußschalter im Untersuchungsraum ausgelöst, ebenso - über einen weiteren Fußschalter - ein Einzelscan (eine Schicht). Um die Lage der Nadel zu kontrollieren, wird der Tisch schnell - und ohne Steuerung von der Konsole - aus der Gantry und wieder in die Gantry bewegt, so dass ein erneuter Einzelscan ausgelöst werden kann.

Überraschenderweise hat sich herausgestellt, dass die erfindungsgemäße Vorgehensweise in der Summe der Veränderungen - Licht, Einzelscan auf Fußdruck, manueller Tisch mit Positionsanzeige und sofortige Bildkontrolle - eine Untersuchungsmodalität schafft, durch die Biopsien sicherer durchgeführt werden können und die zu verkürzten Untersuchungszeiten führt: Da der Patient schnell per Hand aus der Gantry und wieder in sie hineingefahren werden kann - ohne das Untersuchungsprogramm verlassen zu müssen - wird der Zeitbedarf erheblich reduziert. Das aufgenommene Bild wird - damit der Arzt den Raum zur Betrachtung des Bildes nicht verlassen muss - auf einem im Gantryraum stehenden Monitor für den Untersucher sichtbar.

Ein weiterer Vorteil liegt in der Verringerung der Strahlenbelastung des Patienten, da durch die erfindungsgemäß erreichte exakte Tischposition unnötige Scans zum Suchen der Nadel' vermieden werden. Während des Eingriffs bleibt das medizinische Personal im Untersuchungsraum, so dass es positiv auf die Psyche des Patienten einwirken kann.

Die Anwendung der Vorrichtung stellt - neben der Ersparnis von Zeit - eine Vermehrung der technischen Möglichkeiten dar und weist einen weiteren neuen Weg in der Computertomographie-Durchieuchtung für Interventionen. Weitere Vorteile liegen in einer geringeren Strahlenexposition durch eine Reduzierung unnötiger Scans, in einer höheren Sensitivität und indirekt auch in weniger Komplikationen - was in erster Linie durch die unter CT-Durchleuchtung nicht mehr erforderliche häufige Manipulation bei der Nadelkorrektur hervorgerufen wird.

Bei konventioneller Röntgendurchleuchtung werden kontinuierliche Projektionsbilder angefertigt, die eine räumliche Zuordnung nicht gestatten. Dabei wird unter laufender Bestrahlung eine Nadel an den Zielort geführt, soweit dieser in der Projektion überhaupt erkennbar ist. Die Hand des Untersuchers befindet sich überwiegend im direkten Strahlengang. Eine Bewegung des Patiententisches - prinzipiell bei den meisten Durchleuchtungsgeräten möglich - erfolgt jedoch nicht, da unter Röntgensicht punktiert wird und das dargestellte Bild Flächen von 5*5 bis 35*35 cm abbildet. Insofern liegt der Erfindung ein Prinzip zugrunde, das sich von der Einzelscanauslösung und der manuellen Tischbewegung unterscheidet.

Ein Computertomograph mit Handbetrieb ist nicht bekannt, da dies wegen der normalerweise erforderlichen Schichtuntersuchung in definierten Schichtabständen unmöglich ist und keine klinische Relevanz hat. Die Kombination eines schwimmenden Tisches mit einer Schnittbildmodalität ist neu. Sie hat auch nur für Biopsien unter CT-Sichtkontrolle Sinn.

Die Erfindung soll anhand eines Ausführungsbeispieles näher erläutert werden.

### Ausführungsbeispiel

Der Patiententisch wird mit einem Handgriff ausgestattet, der es erlaubt, den Tisch nach mechanischer Entkopplung von der zentralen Steuerung in eine beliebige Position zu bringen. Die digitale Anzeige der Tischposition bleibt davon unbeeinflusst. Nach üblicher Vorbereitung des Patienten kann nun der Arzt nach Einschalten des Lichtvisiers über einen zusätzlich installierten Fußschalter den Tisch exakt positionieren. Anschließend wird über einen zweiten Fußschalter im Untersuchungsraum ein Einzelscan (eine Schicht) ausgelöst. Das Bild erscheint vier Sekunden später auf einem Monitor, durch Ausschalten aller zusätzlichen Filter und Bildnachbearbeitungen nach einer Sekunde. Das aufgenommene Bild wird auf einem im Gantryraum stehenden Monitor für den Untersucher sichtbar.

Der Arzt bleibt im Untersuchungsraum und kann während des Eingriffs positiv auf die Psyche des Patienten einwirken. Muss die Lage der Nadel kontrolliert werden, kann der Tisch schnell und ohne Steuerung von der Konsole aus der Gantry und wieder in die Gantry bewegt werden. Anschließend kann ein erneuter Einzelscan ausgelöst werden.

## Patentansprüche

1. Vorrichtung zur Computertomographie(CT)-Durchleuchtung für Interventionen, bestehend aus der Kombination eines schwimmenden Patiententisches mit einer Schnittbildmodalität für Biopsien unter CT-Sichtkontrolle, **dadurch gekennzeichnet, dass** sie einen mit einem Handgriff versehenen schwimmenden Patiententisch eines Computertomographen zur mechanischen Einstellung einer beliebigen Position des Tisches nach Entkopplung vom zentralen Steuerungssystem und Fußschalter zum Auslösen eines Lichtvisiers zum einen und eines Einzelscans zum anderen enthält.

2. Vorrichtung nach Anspruch 1, **dadurch gekennzeichnet, dass** sie im Gantryraum einen Monitor enthält, der das aufgenommene Bild für den Untersucher sichtbar macht.

## Claims

1. An apparatus for computed tomographyradioscopy (CT-radioscopy) for interventions, comprising the combination of a floating patient table with a sectional drawing modality for biopsies under a visual CT check, **characterized in that** said apparatus comprises a floating patient table of a computer-tomography device, which floating table is provided with a handle for mechanically adjusting a desired position of the table after decoupling from the central control system, and foot switches for triggering a light sight, on the one hand, and a single scan, on the other hand.

2. The apparatus according to claim 1, **characterized in that** said apparatus comprises a monitor in the gantry space, which monitor visualizes the taken image to the examining person.

## Revendications

1. Dispositif de dépistage par tomographie informatisée (CT screening) nécessaire pour des interventions, comprenant une combinaison d'une table flottante pour patient avec une modalité d'image partagée pour des biopsies sous contrôle visuel CT, **caractérisé en ce que** ledit dispositif comprend une table flottante pour patient d'un tomographe informatisé, ladite table flottante étant pourvue d'une poignée pour le réglage mécanique d'une position désirée de la table après une déconnexion du système de contrôle central, et d'interrupteurs à pédale pour déclencher un viseur lumineux, d'une part, et un scan unique, d'autre part.

2. Dispositif selon la revendication 1, **caractérisé en ce que** ledit dispositif comporte dans l'espace du bâti un moniteur qui permet à l'examinateur de voir l'image prise.
